# EUROPEAN PATENT APPLICATION

(11) **EP 4 385 489 A1**
(43) Date of publication of application: **19.06.2024**
(21) Application number: 23211981.8
(22) Date of filing: 24.11.2023
(51) Int. Cl.: A61K 8/42, A61K 8/60, A61K 8/9794, A61Q 7/00

(54) **HAIR CARE COMPOSITION AND BANANA FLOWER EXTRACT AND METHOD FOR HAIR CARE USING THE SAME**

(30) Priority: 16.12.2022 TW 111148602
(71) Applicant: TCI Co., Ltd., Taipei 11494 (TW)
(72) Inventor: LIN, Yung-Hsiang, 11494 Taipei (TW); LIN, Huan-You, 11494 Taipei (TW)
(74) Representative: Viering, Jentschura & Partner mbB Patent- und Rechtsanwälte

(57) **Abstract**

A method for hair care is provided, including administering a composition to a subject in need thereof. The composition includes an effective amount of banana flower extract and a carrier, where the banana flower extract is compound I shown in formula (I) below, compound II shown in formula (II) below, or a combination thereof: 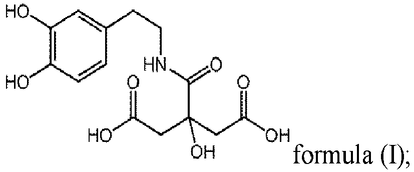 and

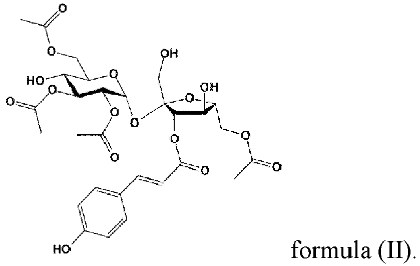

The banana flower extract can be used for increasing hair root diameter sizes, enhancing hair follicle robustness, reducing hair loss, inhibiting dihydrotestosterone (DHT) generation, promoting hair growth, and increasing hair follicle cell proliferation.

## Description

### BACKGROUND

### Technical Field

The present invention relates to a banana flower extract and use thereof, and in particular relates to use of the banana flower extract for hair care.

### Related Art

Bananas are very popular tropical fruit. Fresh bananas can be consumed directly and can also be made into various desserts. At present, bananas are planted on about 10,000 hectares Taiwan. The production is abundant, which can not only meet domestic sales, but also provide export sales. Flowers of the bananas include a male flower at the forefront end, as well as a neutral flower and a female flower. Since only the ovary at the bottom of the female flower grows up to bear a fruit, and the male flower is generally cut off for fear of competing for the nutrients of the banana fruit. The male flowers that are cut off are also referred to as a banana flower.

Alopecia androgenetica is a common cause of hair loss. It is related to genetic constitution and hormones. Testosterone in human bodies will be converted into dihydrotestosterone (DHT) under the action of reductase. Hairs will gradually become thinner and lose under the influence of DHT. Over time, even hair follicles will shrink.

Although a common therapeutic drug Propecia has the effect of avoiding the deterioration of alopecia androgenetica, it also has side effects such as hypaphrodisia, erectile dysfunction, and infertility.

### SUMMARY

In view of this, in order to find a treatment without side effects, and also to develop other application for discarded banana flowers, a hair care and a banana flower extract and use thereof in preparation of the hair care composition are provided.

In some embodiments, a hair care composition includes an effective amount of banana flower extract and a carrier, where the banana flower extract is compound I shown in formula (I), compound II shown in formula (II), or a combination thereof.

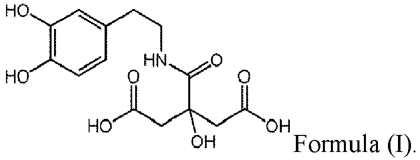

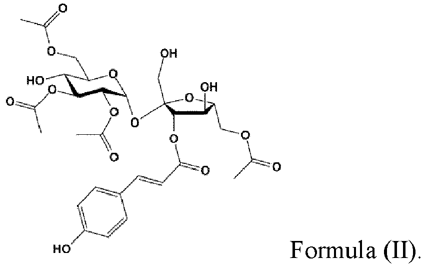

Here, the compound I is *N-*β-citroyldopamine, and the compound II is 6,2',3',6'-O-tetraacetyl-3-*O*-trans-*p*-coumaroylsucrose.

In some embodiments, a banana flower extract is compound I shown in formula (I), compound II shown in formula (II), or a combination thereof.

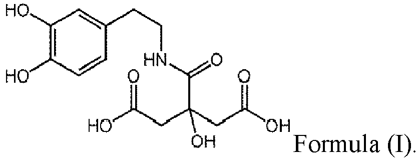

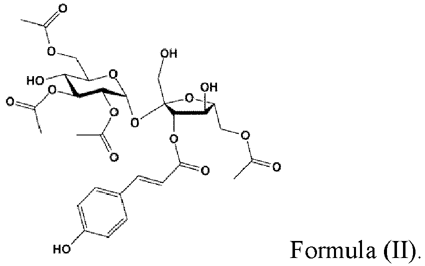

In some embodiments, a banana flower extract is used for preparing a hair care composition, where the banana flower extract is obtained by extracting stamens of the banana flowers through a water extraction step.

In some embodiments, the banana flower extract is obtained by extracting the stamens of the banana flowers through the water extraction step, a re-extraction step, and an elution step. In some embodiments, the re-extraction step is to perform, in the presence of water and n-butanol as solvents, liquid-phase partition extraction on banana flower water extraction liquid obtained in the water extraction step to obtain an n-butanol fractionation layer. The elution step is to perform elution on the n-butanol fractionation layer by using water and a methanol solution of mixed water and methanol.

In some embodiments, the banana flower extract has the effect of increasing hair root diameter sizes.

In some embodiments, the banana flower extract has the effect of enhancing hair follicle robustness.

In some embodiments, the banana flower extract has the effect of reducing hair loss.

In some embodiments, the banana flower extract has the effect of inhibiting dihydrotestosterone (DHT) generation.

In some embodiments, the banana flower extract has the effect of promoting hair growth.

In some embodiments, the banana flower extract has the effect of increasing hair follicle cell proliferation.

In some embodiments, the banana flower extract is compound I shown in formula (I), compound II as shown in formula (II), or a combination thereof.

In summary, the banana flower extract in any embodiment can be used for preparing the hair care composition. In some embodiments, the banana flower extract may have at least one of the following effects: increasing hair root diameter sizes, enhancing hair follicle robustness, reducing hair loss, inhibiting dihydrotestosterone (DHT) generation, promoting hair growth, and increasing hair follicle cell proliferation.

### BRIEF DESCRIPTION OF THE DRAWINGS

FIG. 1 is a result diagram of a banana flower extract inhibiting dihydrotestosterone content in an embodiment.
FIG. 2 is a result diagram of a banana flower extract promoting hair follicle cell proliferation in an embodiment.
FIG. 3 is a result diagram of a banana flower extract promoting hair follicle cell proliferation in another embodiment.
FIG. 4 is a result diagram of a relative average hair root diameter of human testing.
FIG. 5 is a result diagram of hair follicle stability of human testing.
FIG. 6 is a result diagram of relative average hair loss of human testing.

### DETAILED DESCRIPTION

As used herein, "banana flower" refers to the flower of a plant banana.

In some embodiments, a banana flower extract can be extracted from banana flowers of *Musa sapientum L., Musa spp.AAB Silk, Musa spp.ABB Bluggoe, Musa spp.AAA Robusta, Musa spp.AAB Bluggoe,* or *Musa paradisiacal.*

In some embodiments, the banana flowers used for extracting the banana flower extract may be stamens of the banana flowers. The banana flowers may be fresh stamens, dried stamens, or frozen stamens.

In some embodiments, the banana flowers used for extracting the banana flower extract may be intact banana flowers or banana flowers obtained after physical processing procedures such as chopping, dicing, milling, grinding, or other ways that alter the size and physical integrity of raw materials.

In some embodiments, the extraction step of the banana flower extract include: a water extraction step. In some embodiments, the water extraction step of the banana flower extract further includes: a re-extraction step and an elution step.

In the water extraction step, the stamens of the banana flowers are extracted with water to obtain banana flower water extraction liquid.

In the re-extraction step, in the presence of water and n-butanol as solvents, liquid-phase partition extraction is performed on the banana flower water extraction liquid to obtain an n-butanol fractionation layer.

In the elution step, elution is performed on the n-butanol fractionation layer by using water and a methanol solution of mixed water and methanol.

In some embodiments, the water extraction step refers to heating the water to a fixed temperature range, then adding the banana flowers into the water, mixing, and maintaining the above temperature for a fixed period of time to prepare the banana flower water extraction liquid. In some embodiments, the fixed temperature range refers to a range of 30°C to 65°C, 40°C to 60°C, or 45°C to 55°C. In some embodiments, the fixed period of time refers to 50 min to 100 min. For example, the banana flowers are maintained at 50°C for 50 min after being mixed with water of 50°C and extracted to prepare the banana flower water extraction liquid.

In some embodiments, in the water extraction step, the weight ratio of the water to the banana flowers is 2-11 : 1-4. For example, the weight ratio of water: banana flowers is 4 : 1.

In some embodiments, in the water extraction step, primary extraction liquid obtained by extracting the stamens of the banana flower with the water can undergo a centrifugation step and/or a filtration step to obtain the banana flower water extraction liquid. The centrifugation step refers to centrifuging the primary extraction liquid to take its supernatant. In some embodiments, the filtration step refers to passing the supernatant (or primary extraction liquid) obtained from the centrifugation step through a sieve to remove solids by filtration from a solution to obtain a filtrate. For example, the sieve may be a 350-mesh sieve. In other words, the banana flower water extraction liquid may be the aforementioned primary extraction liquid, the supernatant obtained by the centrifugation step, or the filtrate obtained by the filtration step.

In some embodiments, in the re-extraction step, the ratio of the n-butanol to the water may be 3 : 1.

In some embodiments, the elution step may be performing elution twice. First elution may be performing bioassay guided fractionation with a third-order linear gradient of water and methanol on the n-butanol fractionation layer to obtain a pure water fraction and a methanol water solution fraction. Second elution may be performing reverse phase-medium pressure liquid chromatography with a linear elution gradient of pure water to 50% methanol solution on the water fraction, and reverse phase-medium pressure liquid chromatography with a linear elution gradient of pure water to 100% methanol on the methanol water solution fraction.

In some embodiments, the banana flower extract is compound I shown in formula (I) below, compound II as shown in formula (II) below, or a combination thereof: 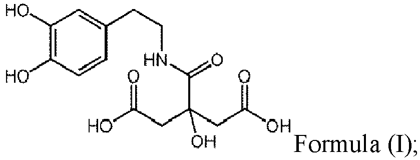 and

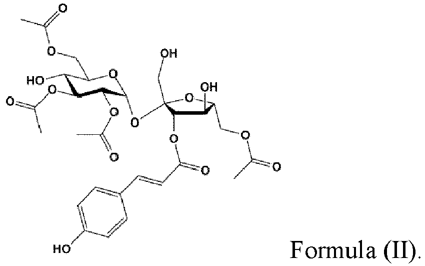

The compound I is *N*-β-citroyldopamine, and the compound II is 6,2',3',6'-O-tetraacetyl-3-*O*-*trans*-*p*-coumaroylsucrose.

In some embodiments, the banana flower extract may be banana flower water extraction liquid, the compound I, the compound II, or a combination of the compound I and the compound II.

In some embodiments, the effective amount of the banana flower extract is 1 g/day.

In summary, the banana flower extract can be used for preparing a hair care composition.

In some embodiments, the aforementioned composition may be a health care product. In other words, the health care product includes an effective amount of banana flower extract.

In some embodiments, the aforementioned health care composition can be manufactured into a dosage form suitable for intestinal or oral administration by using technologies well known to those skilled in the art. These dosage form include, but are not limited to, tablets, troches, lozenges, pills, capsules, dispersible powder or granules, solutions, suspensions, emulsions, syrup, elixirs, slurry, and the like.

In some embodiments, the aforementioned health composition can be manufactured into a dosage form suitable for parenteral or topical administration by using technologies well known to those skilled in the art. These dosage forms include, but are not limited to: injections, sterile powder, external preparations, and the like. In some embodiments, the health care composition can be administered by parenteral routes selected from the group consisting of subcutaneous injection, intradermal injection, intradermal injection, and intralesional injection.

In some embodiments, the health care composition may further include a pharmaceutically acceptable carrier widely used in food manufacturing technologies. For example, the pharmaceutically acceptable carrier may include one or more of the following reagents: a solvent, a buffer, an emulsifier, a suspending agent, a decomposer, a disintegrating agent, a dispersing agent, a binding agent, an excipient, a stabilizing agent, a chelating agent, a diluent, a gelling agent, a preservative, a wetting agent, a lubricant, an absorption delaying agent, a liposome, and the like. The selection and number of these reagents falls within the scope of the professional and routine skills of those familiar with the art.

In some embodiments, the pharmaceutically acceptable carrier includes a solvent selected from the group consisting of water, normal saline, phosphate buffered saline (PBS) and an aqueous solution containing alcohol.

In some embodiments, the aforementioned health care composition may be an edible composition. In some embodiments, the edible composition can be made into a food product or may be a food additive, which means that the food additive is added by a conventional method in food material preparation to prepare the food product, or added in the production process of the food product. Here, the food product may be formulated with edible materials into a product for ingestion by human or animals.

In some embodiments, the food product may be, but is not limited to, beverages, fermented foods, bakery products, health foods, and dietary supplements.

### Example 1: Preparation of Banana Flower Extract

Firstly, bananas (scientific name: Musa paradisiacal) produced in Taiwan were employed, and their stamens, i.e., banana flowers were taken.

Next, water, used as a solvent, was heated to 50 ± 5°C, and then the banana flowers were added. The banana flowers and the water had a weight ratio of 1:4. The banana flowers were maintained at 50 ± 5°C after being mixed with water and extracted for 50 min to obtain primary extraction liquid.

Subsequently, the primary extraction liquid was then sieved through a 350-mesh sieve and then its filtrate was taken to obtain banana flower water extraction liquid.

Next, 10 L of the banana flower water extraction liquid mentioned above was taken, and liquid-phase partition extraction was performed by using n-butanol and water at a ratio of 3:1 to obtain n-butanol layer extraction liquid and water layer extraction liquid. Here, highly polar substances in the banana flower water extraction liquid would remain in n-butanol to form the n-butanol layer extraction liquid, and water-soluble substances in the banana flower water extraction liquid would remain in the water to form the water layer extraction liquid.

Next, the n-butanol layer extraction liquid was concentrated under reduced pressure and dried to obtain 24.6 g of n-butanol fraction (BUF). Next, the water layer extraction liquid was concentrated under reduced pressure and dried to obtain 198.7 g of water fraction (WF).

Based on this, it can be calculated that a total of 223.3 g of powdery extract can be obtained by fractionating and extracting 10 L of banana flower extraction liquid, with 11% of n-butanol fraction (BUF) and 89% of water fraction (WF).

Next, by employing bioassay guided fractionation, 20 g of n-butanol fraction was taken for macroporous resin column chromatography (Diaion HP-20 column chromatography 60×5 cm). By employing pure water, a methanol solution (methanol/pure water = 50/50), and methanol (100%) as elution solutions, elution was performed sequentially to obtain three fractions: a BU1 fraction, a BU2 fraction, and a BU3 fraction.

The BU1 fraction was fractionated by using reverse phase-medium pressure liquid chromatography (RP-MPLC), and was eluted sequentially with water to 50% methanol solution (elution gradient is linear gradient from pure water for 0 min to 50% methanol solution for 80 min). Subsequently, thin-layer chromatography was used, with detection wavelengths of 210 and 280 nm, and the flow rate of 20 mL/min. Every 30 mL was collected to obtain a collection fraction. A total of 54 collection fractions were obtained. Eluents with similar results were combined to obtain five subfractions. The five subfractions were referred to as a BU1-1 subfraction, a BU1-2 subfraction, a BU1-3 subfraction, a BU1-4 subfraction, and a BU1-5 subfraction.

Then, the BU1-3 subfraction was fractionated and purified by HPLC (detection wavelength of 210 nm), and was purified by reverse phase C18 column to obtain compound I by taking 20% methanol as a mobile phase. It was confirmed by ¹H, ¹³C, 2D-NMR (including HSQC, HMBC, COSY, NOESY) and HR-LC-ESI-MS/MS (measured estimated molecular weight *m*/*z* 350.0845 [M+Na]⁺, theoretical estimated molecular formula C₁₄H₁₆NO₈, theoretical estimated molecular weight *m*/*z* 350.0840, and actual molecular formula C₁₄H₁₇NO₈), the compound I was *N*-β-citroyldopamine having a chemical structure of formula (I) below.

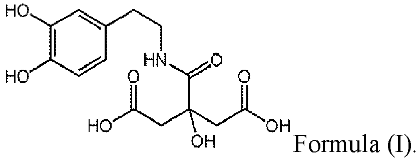

The BU2 fraction was fractionated by using reverse phase-medium pressure liquid chromatography (RP-MPLC), and was eluted sequentially with water to methanol solution (elution gradient was linear gradient from pure water for 0 min to 100% methanol for 120 min). Subsequently, thin-layer chromatography was used, with detection wavelengths of 210 nm and 280 nm, and the flow rate of 20 mL/min. Every 30 mL was collected to obtain a collection fraction. A total of 80 collection fractions were obtained. Eluents with similar results were combined to obtain ten subfractions which were a BU2-1 fraction, a BU2-2 fractions, a BU2-3 fraction, a BU2-4 fractions, a BU2-5 fraction, a BU2-6 fraction, a BU2-7 fraction, a BU2-8 fraction, a BU2-9 fraction and a BU2-10 fraction, respectively.

Then, the BU2-8 subfraction was purified by HPLC (detection wavelength of 310 nm), and was purified by reverse phase C18 column to obtain compound II by taking 45% methanol as a mobile phase. It was confirmed by ¹H, ¹³C, 2D-NMR and HR-LC-ESI-MSMS (measured estimated molecular weight *m*/*z* 655.1888 [M+H]⁺, theoretical estimated molecular formula C₂₉H₃₅O₁₇, theoretical estimated molecular weight *m*/*z* 655.1880, actual molecular formula C₂₉H₃₆O₁₇), the compound II was 6,2',3',6'-O-tetraacetyl-3-*O*-*trans*-*p*-coumaroylsucrose and has the chemical structure of formula (II) below.

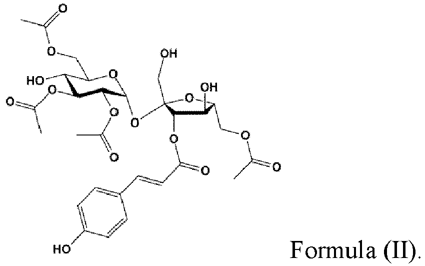

Here, the banana flower water extraction liquid, the compound I and the compound II were the banana flower extracts.

### Example 2: Test of Banana Flower Extract to Inhibit Dihydrotestosterone

A significant increase in the production of dihydrotestosterone (DHT) in hair follicles of the forehead can be observed in young bald female patients. If the dihydrotestosterone can be inhibited, the effect of caring hair follicles and promoting hair growth is achieved. In this test, whether the content of the dihydrotestosterone in human prostate cells treated with different concentrations of banana extract was reduced or not was measured, and a common therapeutic drug Propecia for alopecia androgenetica was used as control.

### Material and Device Description:

Cell line: Human prostate cell LNcap (purchased from BCRC; access number Cat. 60088), hereinafter referred to as LNcap cell.

Cell medium (human prostate cell LNcap cell medium): RPMI 1640 Medium (RPMI), powder (Gibco; Cat.31800-022), 10% fetal bovine serum (FBS) (Gibco; Cat.10437-028) and 1% Antibiotic-Antimycotic(AA) (Gibco; Cat.15240-062).

Reagent: 1XDPBS (Gibco, Cat. 14200-075), trypsin (1 X Trypsin-EDTA, purchased from Thermo, Cat. FNN0011), Propecia (also referred to as Finasteride, purchased from Sigma; Cat. F1293), Testosterone (purchased from Sigma; Cat. T1500), and cell lysis buffer (purchased from RIPA Lysis and Extraction Buffer (Thermo #89900).

Detection kits: dihydrotestosterone ELISA Reagent Detection Kits (USCN; CEA443Ge).

### Test Procedure:

Firstly, LNcap cells were implanted into a 6-well cell culture plate in a density of 1 × 10⁵ LNcap cells and cultured at 37°C in a 5% carbon dioxide incubator for 24 h.

The cultured LNcap cells were divided into an experimental group A, an experimental group B, a control group and a blank group. Here, 10 µg/mL compound I prepared in Example 1 was added into the experimental group A, 5 µg/mL compound I was added into the experimental group B, 20 µg/mL Propecia was added into the control group, and only a cell medium was added into the blank.

Next, 10 mg/mL testosterone was added into each group and placed in a carbon dioxide incubator at 37°C and interacted for 2 h.

After removing the supernatant from each group, the culture plate was washed twice with 1XDPBS. 200 µL of trypsin was added, and interacted with the LNcap cells in a 5% carbon dioxide incubator at 37°C for 3 min. The cell culture plate was gently patted to suspend the LNcap cells.

Subsequently, 600 µL of cell medium was added to each well to terminate the action of the trypsin. Cell fluid in each well was then collected and placed in a 1.5 mL microcentrifuge tube. The microcentrifuge tube was centrifuged at 400 xg for 5 min. After the supernatant in the microcentrifuge tube was removed, 200 µL 1X DPBS was added into the tube to wash the LNcap cells. After the microcentrifuge tube was centrifuged for 5 min again, the supernatant was removed.

Next, a 200 µL 1X cell lysis buffer was added into the tube to lyse the LNcap cells. Centrifugation was performed at 4°C at 13,000 rpm for 5 min, and the supernatant was collected and stored in a 1.5 mL microcentrifuge tube. Finally, the dihydrotestosterone content in the LNcap cells was detected by using a dihydrotestosterone ELISA kit. (Detailed operations as per original factory operation manual)

### Test result:

Here, whether two groups compared have a statistically significant difference or not is analyzed with a single-tailed student t-test to obtain their p-values. Moreover, in the figure, "*" represents that the p-value is less than 0.05, and "**" represents that the p-value is less than 0.01. The more the "*", the more significant the statistical differences.

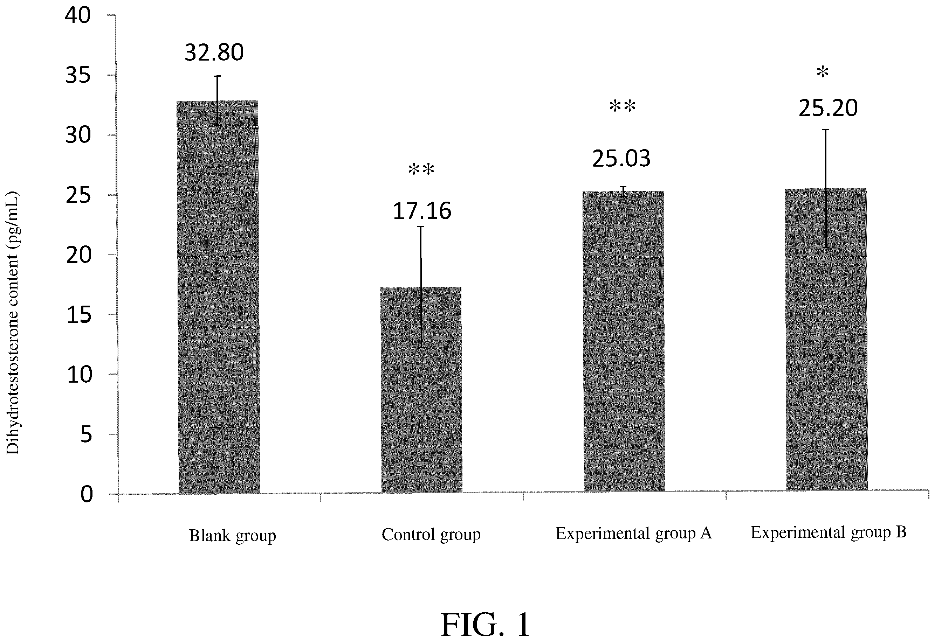

Refer to FIG. 1, the dihydrotestosterone content in the blank group is 32.8 pg/mL, the dihydrotestosterone content in the control group is 17.16 pg/mL, the dihydrotestosterone content in experimental group A was 25.03 pg/mL, and the dihydrotestosterone content in experimental group B is 25.20 pg/mL. It can be seen that whether the experimental group A or the experimental group B using Propecia or banana extract has statistically significant differences relative to the blank group. That is, the banana extract has the effect of inhibiting the dihydrotestosterone.

Furthermore, the efficacy of banana extract B at a concentration of 5 µg/mL was significantly different from that of the blank group, and the banana extract B at a concentration of 10 µg/mL can have a significant efficacy comparable to that of the commercially available drug Propecia^{®}, with the p-value being less than 0.01. From this, it can be seen that the banana flower extract can be used for hair care.

### Example 3: Test of Banana Flower Extract to Promote Hair Follicle Cell Proliferation

Hair follicle cells are the foundation of hair. In hair follicle cells at the growth phase, cells in hair follicle bulbs of the hair root can divide once a day. If the proliferation of the hair follicle cells is promoted, the effect of promoting hair growth can be achieved. In this test, the proliferation of human follicle dermal papilla cells treated with the compound I was examined.

### Material and Device Description:

Cell line: Human Follicle Dermal Papilla Cells (HFDPC) (purchased from PromoCell; Cat. C-12071), hereinafter referred to as HFDPC cells.

Cell medium: Follicle Dermal Papilla Cell Growth Medium (purchased from PromoCell; Cat. C-26501).

Reagents: 10XDPBS (Gibco Company, Cat. 14200-075), and trypsin EDTA (1 X Trypsin EDTA, purchased from the Thermo Company, Cat. FNN0011).

Detection kit: Cell proliferation ELISA reagent detection kit (Click-iT^{™} Plus EdU Flow Cytometry Assay Kits-Alexa Fluor^{™} 488 picolyl azide, 50 tests, brand Invitrogen, model C10632), containing composition C (Dimethyl sulfoxide DMSO), composition D (Click-iT^{™} fixative), composition E (Click-iT^{™} saponin-based permeabilization and wash reagent), composition F (Copper protectant), and composition G (Click-iT^{™} EdU buffer additive).

### Test Procedure:

Firstly, HFDPC cells were implanted into a 6-well cell culture plate in a density of 1 × 10⁵HFDPC cells and cultured at 37°C in a 5% carbon dioxide incubator for 24 h.

The cultured HFDPC cells were divided into an experimental group and a blank group. Here, 10 µg/mL of compound I prepared in Example 1 was added into the experimental group, and only a cell medium was added into the blank group.

Next, 10 uM of composition C was added into each group and placed in a carbon dioxide incubator at 37°C and interacted for 2 h. After removing the cell medium from each group, the culture plate was washed once with 1XDPBS. 200 µL of trypsin was added, and then interacted with the HFDPC cells in a 5% carbon dioxide incubator at 37°C for 5 min. The cell culture plate was gently patted to suspend the HFDPC cells.

Subsequently, after the supernatant was removed, 200 µL of cell medium was added to each well to terminate the action of the trypsin. Cell fluid in each well was collected and placed in a 1.5 mL microcentrifuge tube. The microcentrifuge tube was centrifuged at 400 xg for 5 min. After removing the supernatant in the microcentrifuge tube, washing was performed with 1XDPBS, centrifugation was performed (400 xg for 5 min) and the supernatant was removed. Then 100 µL of composition D was added into the tube and then treated at room temperature in dark for 10 min.

Next, after removing the supernatant in the microcentrifuge tube, washing was performed with 1XDPBS again, centrifugation was performed (400 xg for 5 min) and the supernatant was removed. Then 100 µL of composition E was added into the tube and then treated at room temperature in dark for 15 min. After that, 300 µL of 1% BSA/PBS wash buffer was added, and then centrifugation was performed at 400 xg for 5 min (this step was repeated three times).

The supernatant was removed. Then 100 µL of composition E was added into the tube and then treated at room temperature in dark for 15 min. 100 µl of formulated Click-iT^{™} reactant (formulated according to the operation manual of the cell proliferation ELISA reagent kit) was added into each centrifuge tube and reacted in dark at room temperature for 30 min. 300 µL of 1% BSA/PBS wash buffer was added, and then centrifugation was performed at 400 xg for 5 min. Finally, a fluorescence signal was measured with a flow cytometer (excitation light: 488 nm; scattered light: 527 nm and 590 nm).

### Detection results:

Here, whether two groups compared have a statistically significant difference or not is analyzed with a single-tailed student t-test to obtain their p-values. Moreover, in the figure, "*" represents that the p-value is less than 0.05, and "**" represents that the p-value is less than 0.01. The more the "*", the more significant the statistical differences.

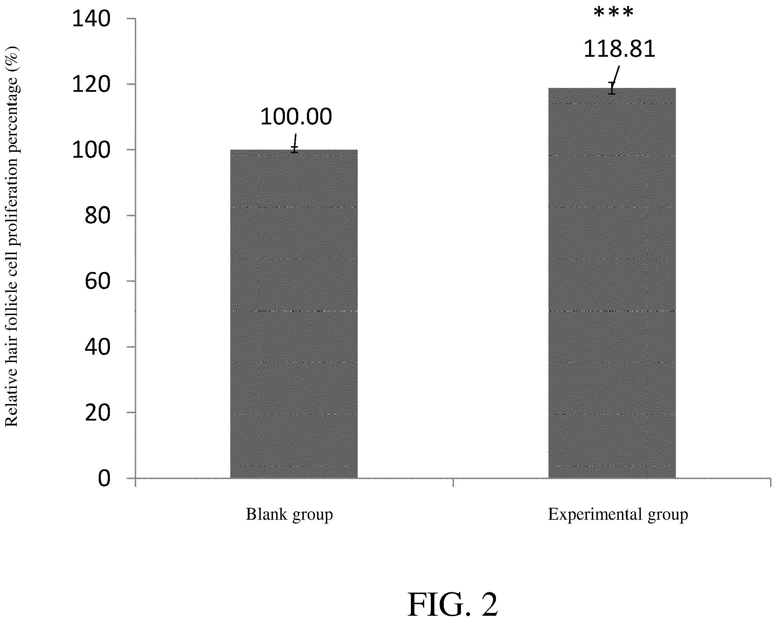

Refer to FIG. 2, when the number of the HFDPC cells in the blank group was considered to be 100%, by conversion, the number of the HFDPC cells in the experimental group was 118.81%. It can be seen that the compound I has a statistically significant difference relative to the blank group. That is, the banana flower extract has the effect of promoting hair follicle cell proliferation.

### Example 4: Test of Banana Flower Extract to Promote Hair Follicle Cell Proliferation

Hair follicle cells are the foundation of hair. In hair follicle cells at the growth phase, cells in hair follicle bulbs of the hair root can divide once a day. If the proliferation of the hair follicle cells is promoted, the effect of promoting hair growth can be achieved. In this test, the proliferation of human follicle dermal papilla cells treated with the compound II was examined.

### Material and Device Description:

Cell line: Human Follicle Dermal Papilla Cells (HFDPC) (purchased from PromoCell; Cat. C-12071), hereinafter referred to as HFDPC cells.

Cell medium: Follicle Dermal Papilla Cell Growth Medium (purchased from PromoCell; Cat. C-26501).

Reagents: 10XDPBS (Gibco Company, Cat. 14200-075), and trypsin EDTA (1 X Trypsin EDTA, purchased from the Thermo Company, Cat. FNN0011).

Detection kit: Cell proliferation ELISA reagent detection kit (Click-iT^{™} Plus EdU Flow Cytometry Assay Kits-Alexa Fluor^{™} 488 picolyl azide, 50 tests, brand Invitrogen, model C10632), containing composition C (Dimethyl sulfoxide DMSO), composition D (Click-iT^{™} fixative), composition E (Click-iT^{™} saponin-based permeabilization and wash reagent), composition F (Copper protectant), and composition G (Click-iT^{™} EdU buffer additive).

### Test Procedure:

Firstly, HFDPC cells were implanted into a 6-well cell culture plate in a density of 1 × 10⁵HFDPC cells and cultured at 37°C in a 5% carbon dioxide incubator for 24 h.

The cultured HFDPC cells were divided into an experimental group, a (positive) control group and a blank group. Here, 100 µM of compound II prepared in Example 1 was added into the experimental group, 20% FBS fetal bovine blood was added into the control group, and only a cell medium was added into the blank group.

Next, 10 µM of composition C was added into each group and placed in a carbon dioxide incubator at 37°C and interacted for 2 h. After removing the cell medium from each group, the culture plate was washed once with 1XDPBS. 200 µL of trypsin was added, and then interacted with the HFDPC cells in a 5% carbon dioxide incubator at 37°C for 5 min. The cell culture plate was gently patted to suspend the HFDPC cells.

Subsequently, after the supernatant was removed, 200 µL of cell medium was added to each well to terminate the action of the trypsin. Cell fluid in each well was collected and placed in a 1.5 mL microcentrifuge tube. The microcentrifuge tube was centrifuged at 400 xg for 5 min. After removing the supernatant in the microcentrifuge tube, washing was performed with 1XDPBS, centrifugation was performed (400 xg for 5 min) and the supernatant was removed. Then 100 µL of composition D was added into the tube and then treated at room temperature in dark for 10 min.

Next, after removing the supernatant in the microcentrifuge tube, washing was performed with 1XDPBS again, centrifugation was performed (400 xg for 5 min) and the supernatant was removed. Then 100 µL of composition E was added into the tube and then treated at room temperature in dark for 15 min. After that, 300 µL of 1% BSA/PBS wash buffer was added, and then centrifugation was performed at 400 xg for 5 min (this step was repeated three times).

The supernatant was removed. Then 100 µL of composition E was added into the tube and then treated at room temperature in dark for 15 min. 100 µl of formulated Click-iT^{™} reactant (formulated according to the operation manual of the cell proliferation ELISA reagent kit) was added into each centrifuge tube and reacts in dark at room temperature for 30 min. 300 µL of 1% BSA/PBS wash buffer was added, and then centrifugation was performed at 400 xg for 5 min. Finally, a fluorescence signal was measured with a flow cytometer (excitation light: 488 nm; scattered light: 527 nm and 590 nm).

### Detection results:

Here, whether two groups compared have a statistically significant difference or not is analyzed with a single-tailed student t-test to obtain their p-values. Moreover, in the figure, "*" represents that the p-value is less than 0.05, and "**" represents that the p-value is less than 0.01. The more the "*", the more significant the statistical differences.

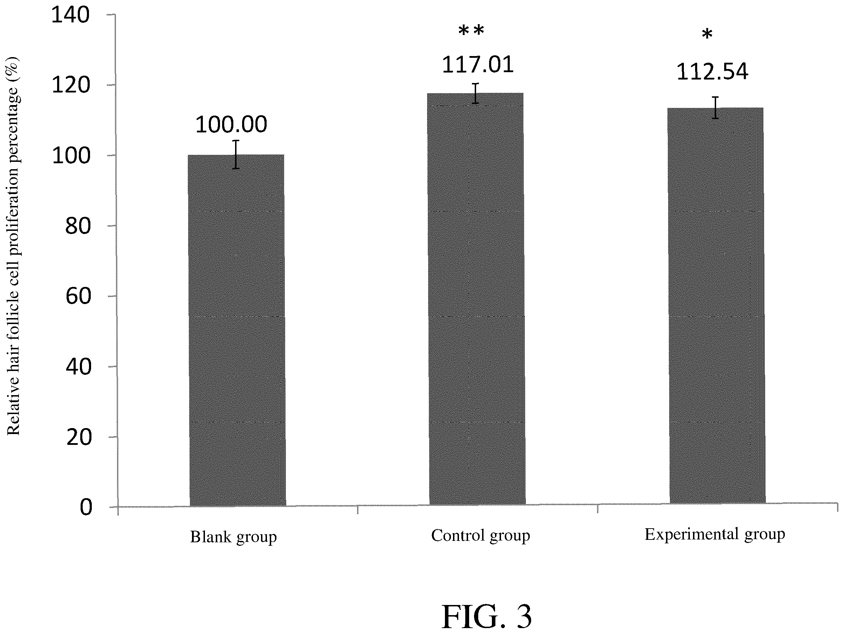

Refer to FIG. 3, when the number of the HFDPC cells in the blank group was considered to be 100%, the number of the HFDPC cells in the control group was 117.01%, and by conversion, the number of the HFDPC cells in the experimental group was 112.54%. It can be seen that the compound II has a statistically significant difference relative to the blank group. That is, the banana flower extract has the effect of promoting hair follicle cell proliferation.

### Example 5: Human Testing of Banana Flower Extract

Subjects: 50 subjects (subjects were divided into a control group with 25 subjects and an experimental group with 25 subjects). Each subject was an adult aged 20 or above.

Test items: hair root diameter, hair follicle stability, and average hair loss by hair washing.

The hair root diameter was measured by using a digital outer diameter micrometer, model Mitutoyo C/N293-100.

The hair follicle stability refers to applying a force to pull up hair (about 60 pieces of hair in each region) in three regions of the frontal bone, temporal bone and occipital bone, and calculating number of hair loss. That is, the less the hair loss, the better the hair follicle stability.

The average hair loss by hair washing refers to the hair loss observed during hair washing.

### Test mode:

The 25 subjects in the experimental group were given daily consumption of powder prepared by drying 1g of banana flower water extraction liquid, while the other 25 subjects in the control group were given daily consumption of a placebo without the banana flower extract daily for 12 consecutive weeks.

Data measured before consumption (i.e., week 0) is referred to as control group 01 and experimental group 01, data measured after 4 weeks of consumption (i.e., week 4) is referred to as control group 02 and experimental group 02, data measured after 8 weeks of consumption (i.e., week 8) is referred to as control group 03 and experimental group 03, and data measured after 12 weeks of consumption (i.e., week 12) is referred to as control group 04 and experimental group 04.

### Test result:

As shown in the figures below, on the basis of the average value of all the subjects and the data at week 0 (considered as 100%), the relative value of each group is obtained by conversion. The standard deviation is calculated by using the STDEV formula of Excel software, whether there is a statistically significant difference or not is analyzed with a single-tailed student t-test to obtain its p-value. Moreover, in the figure, "*" means p-value is less than 0.05, "**" means p-value is less than 0.01, and "***" means p-value is less than 0.001, which represents statistically significant differences. Also, data of the control group at the same number of weeks are compared, and whether there is a statistically significant difference or not is analyzed also with the t-test to obtain its p-value. Moreover, in the figure, "#" means p-value is less than 0.05, "##" means p-value is less than 0.01, and "###" means p-value is less than 0.001, which represents statistically significant differences.

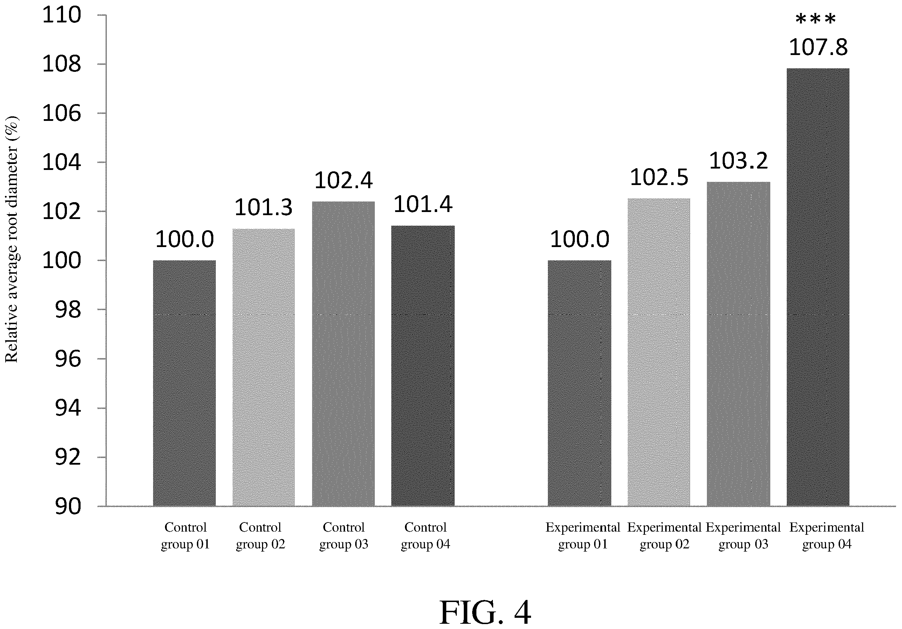

Refer to FIG. 4, after 4 weeks of daily consumption of the banana flower extract, the average hair root diameter of the 25 subjects in the experimental group was increased from 100% at week 0 (experimental group 01) to 102.5% (experimental group 02). After further daily consumption of the banana flower extract to week 8, the average root diameter was increased to 103.2% (experimental group 03). After further daily consumption of the banana flower extract to week 12, the average root diameter significantly was increased to 107.8% (experimental group 04).

With continue reference to FIG. 4, the average hair root diameter of a placebo group was increased from 100% at week 0 (control group 01) to 101.3% (control group 02). After further daily consumption of the banana flower extract to week 8, the average root diameter was increased to 102.4% (control group 03). After further daily consumption of the banana flower extract to week 12, the average root diameter actually was decreased to 101.4% (control group 04).

In general, thinning of hair is one of initial symptoms of alopecia androgenetica. That is, when it is measured that the hair root diameter is getting thicker, alopecia androgenetica begins to alleviate. It can be seen in the experimental group in this test from week 0 to week 12 that the hair root diameter is getting thicker, especially 6.4% higher than that of the control group at week 12. That is, even irrespective of the placebo effect, the root diameter can be significantly increased by daily consumption of the banana flower extract, so that every hair is healthier to achieve the effect of hair care.

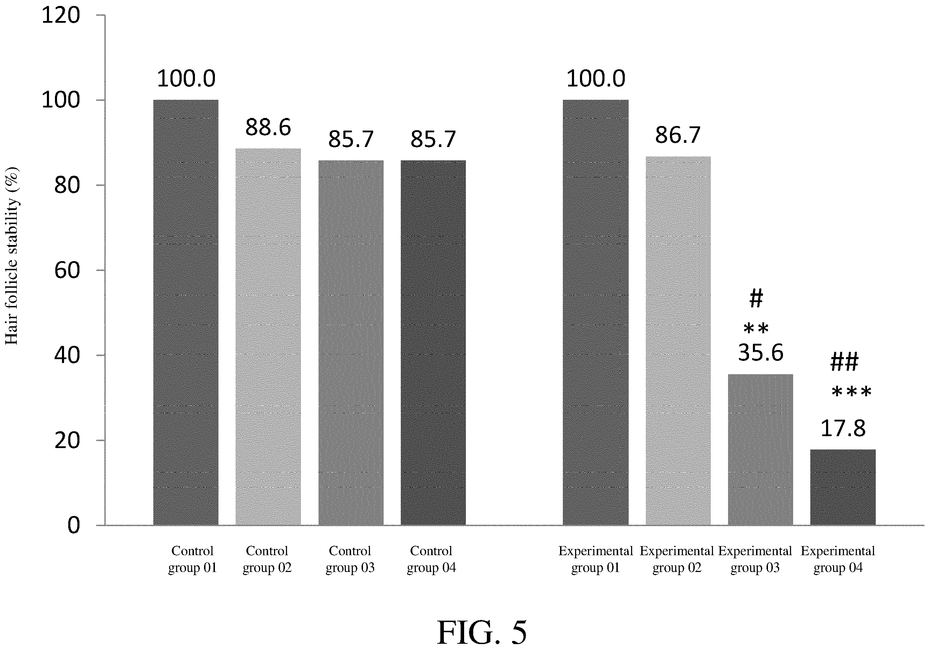

Refer to FIG. 5, after 4 weeks of daily consumption of the banana flower extract, the hair follicle stability of 25 the subjects in the experimental group was decreased from 100% at week 0 (experimental group 01) to 86.7% (experimental group 02). After further daily consumption of the banana flower extract to week 8, the hair follicle stability was decreased to 35.6% (experimental group 03). After further daily consumption of the banana flower extract to week 12, the hair follicle stability significantly was decreased to 17.8% (experimental group 04).

With continue reference to FIG. 5, the hair follicle stability of a placebo group was increased from 100% at week 0 (control group 01) to 88.6% (control group 02). After further daily consumption of the banana flower extract to week 8, the average root diameter was increased to 85.7% (control group 03). After further daily consumption of the banana flower extract to week 12, the hair follicle stability actually was decreased to 85.7% (control group 04).

It can be seen in the experimental group in this test from week 0 to week 12 that the hair loss continues to significantly decrease even if the force is applied to pull the hair, especially 67.9% lower than that of the control group at week 12. That is, even irrespective of the placebo effect, the hair loss due to the fact that the force is applied to pull the hair can be significantly decreased by daily consumption of the banana flower extract, and the higher hair follicle stability is achieved, so that every hair is healthier to achieve the effect of hair care.

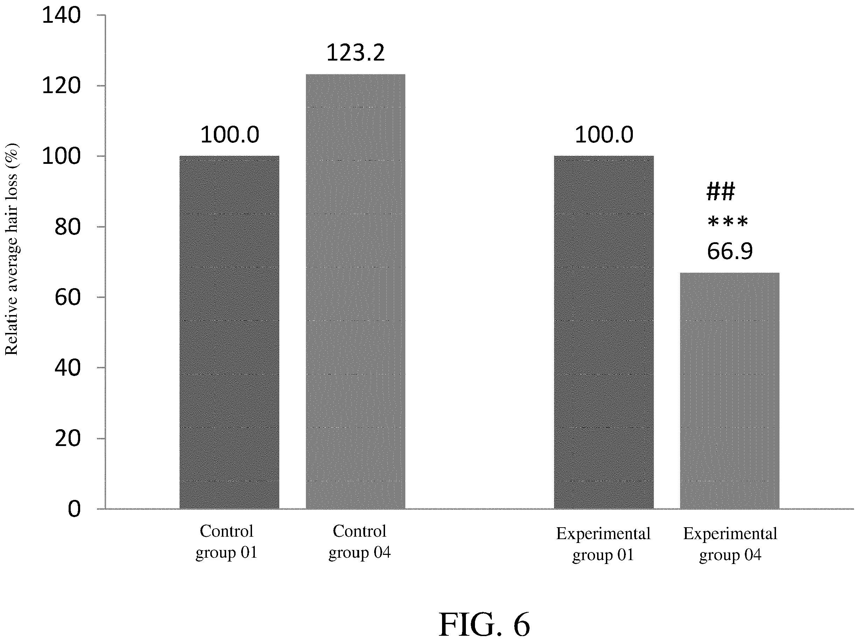

Refer to FIG. 6, after 12 weeks of daily consumption of the banana flower extract, the relative average hair loss of the 25 subjects of the experimental group during hair washing significantly was decreased from 100% at week 0 (experimental group 01) to 66.9% (experimental group 04). With continue reference to FIG. 6, the relative average hair loss of the placebo group actually was increased from 100% at week 0 (control group 01) to 123.2% (control group 04).

It can be seen in the experimental group in this test from week 0 to week 12 that the hair loss continues to significantly decrease even if the force is applied to pull the hair, especially 66.9% lower than that of the control group at week 12. That is, even irrespective of the placebo effect, the hair loss due to the fact that the force is applied to pull the hair can be significantly decreased by daily consumption of the banana flower extract, and the higher hair follicle stability is achieved, so that every hair is healthier to achieve the effect of hair care.

In summary, the banana flower extract in any embodiment can be used for preparing the hair care composition. The banana flower extract in any embodiment may be prepared to increase hair root diameter sizes, enhance hair follicle robustness, reduce the hair loss, inhibit dihydrotestosterone (DHT) generation, promote hair growth, and increase hair follicle cell proliferation.

Although the present invention has been described in considerable detail with reference to certain preferred embodiments thereof, the disclosure is not for limiting the scope of the invention. Persons having ordinary skill in the art may make various modifications and changes without departing from the scope and spirit of the invention. Therefore, the scope of the appended claims should not be limited to the description of the preferred embodiments described above.

## Claims

1. A hair care composition, comprising an effective amount of banana flower extract and a carrier, wherein the banana flower extract is compound I shown in formula (I) below, compound II shown in formula (II) below, or a combination thereof: 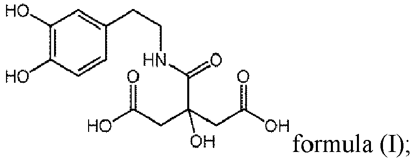 and

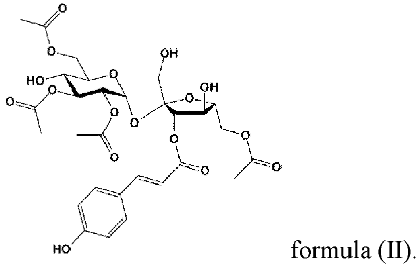

2. A banana flower extract, being compound I shown in formula (I), compound II shown in formula (II), or a combination thereof: 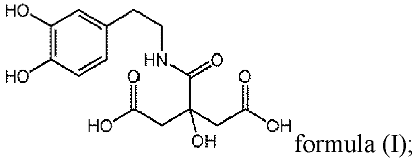 and

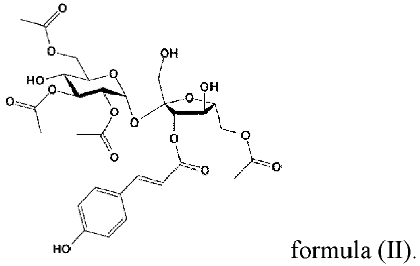

3. A method for hair care, comprising administrating to a subject in need thereof a composition comprising a banana flower extract, wherein the banana flower extract is obtained by extracting stamens of banana flowers through a water extraction step.

4. The method according to claim 3, wherein the banana flower extract has the effect of increasing hair root diameter sizes.

5. The method according to claim 3, wherein the banana flower extract has the effect of enhancing hair follicle robustness.

6. The method according to claim 3, wherein the banana flower extract has the effect of reducing hair loss.

7. The method according to claim 3, wherein the banana flower extract is obtained by extracting the stamens of the banana flowers through the water extraction step, a re-extraction step, and an elution step.

8. The method according to claim 7, wherein the banana flower extract is compound I shown in formula (I), compound II shown in formula (II), or a combination thereof: 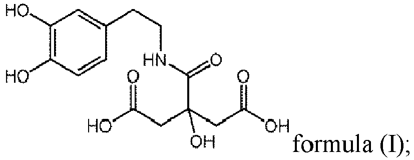 and

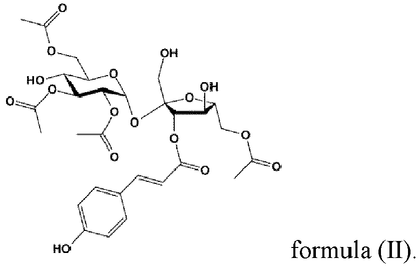

9. The method according to claim 7, wherein the banana flower extract has the effect of inhibiting dihydrotestosterone (DHT) generation.

10. The method according to claim 7, wherein the banana flower extract has the effect of promoting hair growth.

11. The method according to claim 7, wherein the banana flower extract has the effect of increasing hair follicle cell proliferation.
